# EUROPÄISCHE PATENTSCHRIFT

(11) **EP 2 701 904 B1**
(45) Veröffentlichungstag und Bekanntmachung des Hinweises auf die Patenterteilung: **15.07.2020**
(21) Anmeldenummer: 12723841.8
(22) Anmeldetag: 27.04.2012
(51) Int. Cl.: B32B 5/00, B32B 5/04, B32B 5/12, B32B 5/22, B32B 5/26, B32B 7/02, B32B 7/10, B32B 27/00, B32B 27/02, B32B 27/12, B32B 27/24, B32B 27/28, A61F 13/00, A61F 13/532

(54) **ELASTISCHER ABSORBIERENDER HYGIENEARTIKEL FÜR DIE AUFNAHME VON KÖRPERFLÜSSIGKEITEN**
ELASTIC ABSORBENT SANITARY ARTICLE FOR ABSORBING BODILY FLUIDS
ARTICLE HYGIÉNIQUE ABSORBANT ÉLASTIQUE POUR L'ABSORPTION DE FLUIDES CORPORELS

(30) Priorität: 28.04.2011 DE 102011018985
(43) Veröffentlichungstag der Anmeldung: 05.03.2014
(73) Patentinhaber: Evonik Operations GmbH, 45128 Essen (DE)
(72) Erfinder: FENSKE, Wilfried, 8820 Wädenswil (CH)
(74) Vertreter: Evonik Patent Association
(86) Internationale Anmeldenummer: PCT/EP2012/057833
(87) Internationale Veröffentlichungsnummer: WO 2012/146748

(56) Entgegenhaltungen:
- WO-A1-00/35503
- WO-A1-01/34082
- WO-A1-99/49826
- WO-A1-03/106162
- GB-A- 2 181 036
- US-A1- 2003 082 966

## Beschreibung

Die Erfindung betrifft einen elastischen absorbierenden Hygieneartikel für die Aufnahme von Körperflüssigkeiten mit einem biegeweichen Topsheet und einem biegeweichen Backsheet, zwischen denen haftend ein absorbierendes Laminat eingebracht ist, wobei das absorbierende Laminat aus zwei biegeweichen äußeren Lagen besteht, zwischen denen mindestens zwei sich diagonal kreuzende, vorgespannte Lagen von elastischen Fäden haftend eingelagert sind und wobei in dem Laminat diskrete Sektionen von superabsorbierendem Polymer eingelagert sind.

Moderne Hygieneartikel für die Aufnahme von Körperflüssigkeiten, wie Urin, Schweiß, Menstruationsblut oder Wundsekreten basieren grundsätzlich auf einer Schichtung mehrerer Lagen funktioneller biegeweicher Materialien, welche in geeigneter Form die ausgeschiedene Flüssigkeit von der Emissionsstelle aufnehmen, verteilen, speichern und nach außen abschließen. Zusätzlich wird versucht, die Rücknässung zur Haut des Trägers zu minimieren, und durch geeignete elastische Elemente, wie elastische Verschlusssysteme und gezielte Elastifizierung einzelner Bereiche wird eine optimale Anpassung an die Anatomie des Benutzers angestrebt.

Diese Vorgehensweise ist einheitlich bei der Entwicklung von verschiedensten Hygiene- und Medizinartikeln zu finden, wie etwa Babywindeln, Damenbinden, Inkontinenzprodukten, Verbandsmaterialien, klinischem Absorptionsmaterial, Verpackungsmaterial für Lebensmittel, etc.

Es ist grundsätzlich wünschenswert, die Materialien zur Erfüllung dieser Aufgabe nach Anzahl und Menge zu optimieren, um sowohl den Material- und Energieeinsatz bei der Herstellung dieser Produkte, als auch das Volumen des Produktes und damit dessen Lagerungs-, Distributions- und Entsorgungsbedarf ökonomisch und ökologisch vernünftig zu gestalten.

Produkte der oben beschriebenen Funktion bestehen grundsätzlich aus einer der Haut des Trägers zugewandten ersten äußeren Lage (Topsheet) zwecks Hautfreundlichkeit, einer gegenüberliegenden zweiten äußeren Lage (Backsheet) zwecks Absicherung gegen ein unerwünschtes Austreten von Flüssigkeit in die Kleidung oder in die Umgebung, sowie darin eingelagert einen Absorptionskern zur Absorption und Speicherung der Körperflüssigkeit. Dieser Absorptionskernsoll die Funktionen schneller Flüssigkeitsaufnahme, schneller Querverteilung im Produkt und zuverlässige Speicherung der Flüssigkeit in dem Absorptionskern mit dem letztlichen Ziel einer minimalen Rücknässung durch die in dem Absorptionskern aufgenommene Flüssigkeit erfüllen.

Bei den aus der Praxis bekannten Produkten werden die Funktion der Flüssigkeitsaufnahme durch Lagen von schnell flüssigkeitsleitender Materialien, wie etwa leichter, voluminöser Spinnvliese oder Nadelfilze auf Polyester-, Polypropylen- oder Polyethylenbasis, die Funktion der Verteilung der Flüssigkeit in dem Absorptionskern durch Zellstofffasern, Zellulose oder chemisch modifizierte Zellstofffasern ("Curly Fibers")und die Funktion der Speicherung der Flüssigkeit durch superabsorbierende Polymere erzielt. Es liegt in der Natur der Sache, dass sich auf Grund der Mehrfachwirkung der einzelnen Materialien hier die Aufgaben teilweise überschneiden. Auch ist es offensichtlich, dass Materialien, welche Flüssigkeiten gut transportieren und weiterleiten können, diese Transportfunktion üblicherweise in alle Richtungen gleichermaßen erfüllen. Eine gute Transport- und Weiterleitung von Flüssigkeit von der Hautseite weg in Richtung des Absorptionskerns bedingt deshalb regelmäßig eine vergleichbar gute Transport- und Weiterleitungswirkung zurück in Richtung der Hautseite des Trägers, wodurch eine unerwünschte Rücknässung begünstigt wird.

Es wird deshalb als eine Aufgabe der vorliegenden Erfindung angesehen, einen Hygieneartikel der eingangs genannten Gattung so auszugestalten, dass mit geringem Material- und Herstellungsaufwand die Eigenschaften des Hygieneartikels verbessert werden. Dabei soll nach Möglichkeit die Speicherung von Flüssigkeit möglichst schnell und zuverlässig gewährleistet werden und gleichzeitig die Gefahr einer Rücknässung verringert werden.

Diese Aufgabe wird erfindungsgemäß dadurch gelöst, dass die topsheet-seitige äußere Lage des Laminats ein hydrophobes Material ist, welches im Bereich der Verhaftung mit den elastischen Fäden und der zweiten äußeren Lage punktuell oder sektionsweise mechanisch, chemisch oder thermisch flüssigkeitsdurchlässig gemacht wurde. Im Gegensatz zu den aus der Praxis bekannten Hygieneprodukten wird die dem Topsheet und damit der Hautseite des Hygieneprodukts zugewandte äußere Lage des Laminats nicht im Hinblick auf eine rasche Flüssigkeitsaufnahme und Verteilung optimiert, sondern ganz im Gegenteil hydrophob ausgestaltet, um einer unerwünschten Rücknässung entgegenzuwirken, die durch einen Rücktransport der zunächst in dem Laminat aufgenommenen Flüssigkeit zur Hautseite hin verursacht werden könnte. Diese Rücknässung wird durch die hydrophoben Eigenschaften der dem Topsheet zugewandten äußeren Lage des Laminats weitestgehend verhindert. Durch das erfindungsgemäße Laminat wird eine verbesserte Flüssigkeitsaufnahme durch das superabsorbierende Polymermaterial unterstützt.

Gemäß einer vorteilhaften Ausgestaltung des Erfindungsgedankens ist vorgesehen, dass die topsheet-seitige äußere Lage des Laminats eine hohe Sperrwirkung für Flüssigkeiten aufweist.

Zur weiteren Verdeutlichung der Eigenschaften der erfindungsgemäßen Hygieneprodukte dienen die folgenden Beispiele. Bei den dafür durchgeführten Versuchen wurden die folgenden Vliesmaterialien verwendet und untersucht, die nachfolgend nur noch mit der jeweils angegebenen Kurzbezeichnung bezeichnet werden:

| **Kurzbezeichnung** | **Bezeichnung** | **Hersteller** | **Grammatur** | **Eigenschaft** |
|---|---|---|---|---|
| Corovin | PC 5FF-111 | Corovin GmbH; Peine, Deutschland | 17gsm | hydrophob |
| Fibrella | Fibrella 30 | Suominen Nonwovens Ltd.; Nakkila, Finnland | 30gsm | hydrophil |
| Novellin | Novellin 23 | Suominen Nonwovens Ltd.; Nakkila, Finnland | 23gsm | hydrophil |
| Pegas | Pegatex 13 | Pegas nonwovens s.r.o.; Znojmo, Tschechische Republik | 13gsm | hydrophob |

Jeweils mehrere Vliesstoffmuster dieser Vliesmaterialien wurden auf diejenigen Eigenschaften wie beispielsweise Sperrwirkung und Spreitung, bzw. Spreading untersucht, die für die vorliegende Erfindung relevant sind.

### Beispiel 1: Bestimmung der Sperrwirkung

Um in geeigneter Weise die Sperrwirkung eines Vliesmaterials zu bestimmen können wurde für verschiedene Vliesstoffe die Höhe einer auf dem Vliesstoff aufstehenden Flüssigkeitssäule bestimmt.

Als Testapparatur wurde eine durchsichtige Flüssigkeitssteigsäule mit einer Länge von 50 cm und einem Innendurchmesser von 1,2 cm (Außendurchmesser 1,6 cm) verwendet, die mit einer durchgehenden Skalierung in 0,1 cm Schritten versehen ist. Als Testmuster wurden jeweils quadratische Vliesstoffmuster mit 6 cm Kantenlänge verwendet. Als Testflüssigkeit wurde eine 0,9%ige NaCl-Lösung verwendet. Das Vliesstoffmuster wird mittels einer Schlauchklemme oder eines Gummirings am unteren Ende der Flüssigkeitssteigsäule befestigt und dabei derartig orientiert, dass die gegebenenfalls rauhere Seite des Vliesstoffmusters in Richtung der Flüssigkeitssteigsäule zeigt. Die Position des Klemm- bzw. Gummirings ist hierbei 1 cm oberhalb des unteren Rohrendes der Flüssigkeitssteigsäule zu wählen, wobei die Befestigung des Vliesstoffmusters am Rohr derart sein muss, dass es während der Testdurchführung zu keinem Flüssigkeitsaustritt oberhalb des Klemmrings kommt. Mittels einer kalibrierten Pumpe (z.B. Ismatec MCP ISM 404B) wird die Testlösung mit einer Dosierrate von 60g/min in die Flüssigkeitssteigsäule zugegeben. Hierbei ist das untere Ende der Zuleitung der Pumpe 20 cm oberhalb des Vliesstoffmusters zu justieren. Mit dem Start der Pumpe wird auch gleichzeitig die Zeitmessung gestartet. Die Höhe der sich in der Flüssigkeitssteigsäule bilden Überstandes, bzw. die Flüssigkeitspegelhöhe wird beim ersten Durchtropfen von Testflüssigkeit sowie jeweils nach 1, 2, 3, 4, und 5 Minuten abgelesen.

In der nachfolgenden Übersicht sind die durchschnittlichen Pegelhöhen nach 5 Minuten, bzw. 300 Sekunden wiedergegeben, wobei die über jeweils 5 Messungen gemittelten Durchschnittswerte angegeben sind:

| | | |
|---|---|---|
| | Zeit [s] | Ø Höhe [cm] |
| Fibrella | 300 | 0,1 |
| Novellin | 300 | 0,2 |
| Pegas | 300 | 3,3 |
| Corovin | 300 | 11,5 |

Es zeigt sich, dass hydrophobe Vliese wie beispielsweise Corovin oder Pegas eine deutlich höhere Flüssigkeitspegelhöhe als andere Vliese (beispielsweise Fibrella oder Novellin) aufweisen. Die Sperrwirkung der hydrophoben Vliese ist demzufolge erheblich besser als die Sperrwirkung der hydrophilen Vliese. Diese Sperrwirkung der topsheet-seitigen äußeren Lage ist maßgeblich für die geringen Rücknässeigenschaften des erfindungsgemäßen Laminats verantwortlich. Es hat sich überraschender Weise gezeigt, dass entgegen dem Vorurteil der Fachwelt trotz einer guten Sperrwirkung der topsheet-seitigen äußeren Lage eine rasche Flüssigkeitsaufnahme und großflächige Verteilung innerhalb des Laminats ermöglicht werden kann und die Sperrwirkung der topsheet-seitigen äußeren Lage diese zusätzlich geforderten Eigenschaften nicht grundsätzlich ausschließt.

Für die vorliegende Erfindung wird von einer guten Sperrwirkung ausgegangen, wenn die gemessene durchschnittliche Flüssigkeitspegelhöhe mehr als 2 cm beträgt.

Von einer sehr guten Sperrwirkung wird ausgegangen, wenn die gemessene durchschnittliche Flüssigkeitspegelhöhe mehr als 5 cm beträgt. Gemäß einer besonders vorteilhaften Ausgestaltung des Erfindungsgedankens zu Folge ist deshalb vorgesehen, dass die topsheet-seitige äußere Lage des Laminats eine sehr gute Sperrwirkung aufweist, also eine Flüssigkeitssäule von mehr als 5 cm, bevorzugt von mehr als 8 cm und insbesondere bevorzugt von mehr als 10 cm aushält, wenn die vorangehend näher erläuterte Bestimmung der Flüssigkeitspegelhöhen durchgeführt wird.

Um die Verteilung und rasche Aufnahme einer größeren Flüssigkeitsmenge innerhalb des Laminats zu unterstützen ist erfindungsgemäß weiterhin vorgesehen, dass die backsheet-seitige äußere Lage des Laminats ein hydrophiles Material mit guter Flüssigkeitstransporteigenschaft ist.

Die backsheet-seitige äußere Lage des Laminats ist dabei zweckmäßigerweise ein hydrophiles Material mit einer hohen Flächenverteilung eines nahezu punktförmig aufgebrachten Flüssigkeitstropfens.

### Beispiel 2: Bestimmung der Flächenverteilungseigenschaften

Zur Charakterisierung der Flächenverteilungseigenschaften einer Flüssigkeit, die auf ein Vliesmaterial aufgebracht wird, wurde für verschiedene Vliesstoffe die Spreitung, bzw. das Spreading gemäß der nachfolgend beschriebenen Versuchsdurchführung bestimmt. Als Testapparatur wurden ein innerer Kunststoffring mit einem Außendurchmesser von 9 cm (Höhe 8 cm, Innendurchmesser 8,2 cm) sowie ein äußerer Kunststoffring mit einem Innendurchmesser von 9,2 cm verwendet. Als Testmuster wurden quadratische Vliesstoffmuster mit 15 cm Kantenlänge verwendet. Als Testflüssigkeit wurde eine 0,9%ige NaCl-Lösung verwendet, die mit Patentblau (0,8g auf 100g NaCl-Lösung) eingefärbt wurde. Das Vliesstoffmuster wird mittig auf den kleineren Kunststoffring aufgelegt und durch ineinanderschieben der beiden Ringe fixiert. Hierbei ist die gegebenenfalls rauherer Seite des Vliesstoffmusters nach oben orientiert (zur Flüssigkeitsaufgabe hin). Mit einer Eppendorfpipette wird unter einem Winkel von ca. 30° 1 ml der Testlösung vorsichtig mittig auf das Vliesstoffmuster gegeben. Nach einer Wartezeit von 20 min wird gegebenenfalls die auf dem Vliesstoffmuster verbliebene Testflüssigkeit mit einer Pipette abgenommen. Die Vliesstoffmuster werden anschließend bei 30°C für 4h getrocknet. Zur Bestimmung der benetzten Fläche wird das getrocknete Vliesstoffmuster fotokopiert und der benetzte Bereich (erscheint dunkel auf der Fotokopie) durch ausschneiden und wiegen bestimmt (Flächengewicht des Papiers 0,01 g/cm²).

In der nachfolgenden Übersicht ist die durchschnittliche Größe der von der Flüssigkeitsmenge benetzte Fläche des Vliesstoffmusters gemittelt über jeweils 4 Messungen angegeben:

| | Benetzte Fläche [cm²] |
|---|---|
| Fibrella | 57,0 |
| Novellin | 20,5 |
| Corovin | 2,1 |
| Pegas | 1,8 |

Es zeigte sich, dass einige hydrophile Vliesstoffe wie beispielsweise Fibrella oder Novellin eine deutlich bessere Flüssigkeitsverteilungseigenschaft als andere Vliesstoffe wie beispielsweise Corovin oder Pegas aufweisen.

Von einer hohen Flächenverteilung einer auf das Vliesmaterial aufgebrachten Flüssigkeit wird im Zusammenhang mit der vorliegenden Erfindung ausgegangen, wenn die auf das Vliesmaterial aufgebrachte Flüssigkeitsmenge von 1 ml durchschnittlich auf eine benetzte Fläche von mehr als 10 cm² verteilt wird.

Von einer sehr hohen Flächenverteilung wird ausgegangen, wenn die auf das Vliesmaterial aufgebrachte Flüssigkeitsmenge durchschnittlich auf eine benetzte Fläche von mehr als 20 cm² verteilt wird. Gemäß einer besonders vorteilhaften Ausgestaltung des Erfindungsgedankens zu Folge ist deshalb vorgesehen, dass die auf das Vliesmaterial aufgebrachte Flüssigkeitsmenge durchschnittlich auf eine benetzte Fläche von mehr als 20 cm², bevorzugt mehr als 40 cm² und besonders bevorzugt mehr als 55 cm² verteilt wird, wenn die vorangehend näher erläuterte Bestimmung der Flüssigkeitsverteilung durchgeführt wird.

Zur Ermittlung einer besonders vorteilhaften erfindungsgemäßen Ausgestaltung des absorbierenden Laminats wurden Versuche durchgeführt, wobei jeweils die Flüssigkeitsaufnahme und eine Rücknässung für verschiedene Kombinationen von hydrophilen und hydrophoben Materialien für die äußeren Lagen des Laminats bestimmt und untersucht wurden.

### Beispiel 3: Bestimmung der charakteristischen Eigenschaften verschiedener absorbierender Laminate

Es wurde eine größere Anzahl von im Wesentlichen gleichartigen Testmustern für ein saugfähiges Hygieneprodukt mit den erfindungsrelevanten Merkmalen hergestellt, wobei jeweils identisch übereinstimmende Topsheets und Backsheets verwendet wurden. Die darin eingelegten und festgelegten absorbierenden Laminate unterscheiden sich lediglich hinsichtlich der jeweils verwendeten Materialien für die topsheet-seitige äußere Lage und für die backsheet-seitige äußere Lage. Ansonsten stimmen der Aufbau des absorbierenden Laminats einschließlich dessen Abmessungen und des verwendeten superabsorbierenden Polymermaterials identisch überein.

Zur Herstellung der absorbierenden Laminate wurden zwei Stiftschienen von 1200 mm Länge in einem Spannrahmen im Abstand von 160 mm zueinander angeordnet. Auf jeder Stiftschiene ist jeweils eine Reihe von 24 Stiften im Abstand von 45 mm zueinander angeordnet. Ein elastisches Garn (615 dTex, 550 den, Dorlastan) wurde, ohne Vorspannung, anfangend von einem 1. Stift an einem Ende der ersten Stiftschiene schräg zu dem 10. Stift der gegenüberliegenden Stiftschiene geführt, um den seitlich benachbarten 11. Stift dieser gegenüberliegenden Stiftschiene gewunden, schräg zurück zum 2. Stift der ersten Stiftschiene und anschließend zum seitlich benachbarten 3. Stift der ersten Stiftschiene geführt. Dadurch wurden zwei schräg verlaufende parallele Garnabschnitte zwischen den beiden Stiftschienen erzeugt. Ausgehend von dem 3. Stift der ersten Stiftschiene wurde diese Vorgehensweise bis zum vorletzten Stift der gegenüberliegenden Stiftschiene wiederholt und dadurch 14 parallel zueinander und schräg zwischen den beiden Stiftschienen verlaufende Garnabschnitte erzeugt. Im Anschluss daran wurde das elastische Garn zum letzten Stift der gegenüberliegenden Stiftschiene und dann zum letzten Stift der ersten Stiftschiene geführt, um von diesem Stift ausgehend einen gespiegelten Garnverlauf bis zurück zum 1. Stift der gegenüberliegenden Stiftschiene zu erzeugen. Das Garn bildet dann zwei gekreuzte Lagen zwischen den beiden Stiftschienen, die jeweils aus parallel zueinander und mit gleicher Neigung schräg zwischen den Stiftschienen verlaufenden Garnabschnitten bestehen.

Die beiden Stiftschienen werden auf einen Abstand von 390 mm zueinander auseinander gezogen und die Garnabschnitte bzw. das gesamte Garn gespannt. Die beiden gekreuzten Lagen der Garnabschnitte bilden dann ein wabenförmiges Muster, wobei die einzelnen Waben eine Größe von etwa 30 mm x 30 mm aufweisen.

Mit einer Klebepistole (Leimpistole HB 700 von Bühnen mit einer Hotmelt-Düse von Bühnen, Druck etwa 2,5 bar, Leim BOSTIK 2052 FUN von Bostik) wird innerhalb von einer Minute eine Leimmenge von etwa 1,7 g gleichmäßig unter einem Sprühwinkel von 45° auf die gespannten Garnabschnitte gesprüht. An der Unterseite der Garnabschnitte herabhängende Leimfäden werden vorsichtig mit der Hand entfernt.

Auf einer Marmorplatte wird eine erste äußere Lage beispielsweise aus Corovin mit Abmessungen von 300 mm x 500 mm und anschließend eine Lochplatte (250 mm x 500 mm mit 72 Bohrungen mit einem jeweiligen Abstand von 45 mm, bzw. einem Lochmuster 45/405) aus Metall aufgelegt. Die Löcher der Lochplatte sind an die Anordnung der Waben der gekreuzten Lagen der Garnabschnitte angepasst, so dass jeder Wabe etwa mittig ein Loch der Lochplatte zugeordnet ist.

12,5 g eines superabsorbierenden Polymermaterials werden mit einer Laborwaage abgewogen und gleichmäßig mit einem Rakel oder mit einem Spatel in den Löchern der Lochplatte verteilt. Das überschüssige superabsorbierende Polymermaterial wird entfernt und anschließend die Lochplatte abgehoben.

Im Anschluss daran wird der Spannrahmen mit den gekreuzten Lagen der elastischen Garnabschnitte über die erste äußere Lage mit den darauf verteilten Portionen des superabsorbierenden Polymermaterials gestellt, wobei auf eine mittige Anordnung des superabsorbierenden Polymermaterials innerhalb der einzelnen Waben und eine entsprechende Ausrichtung des Spannrahmens geachtet werden muss.

Eine zweite äußere Lage beispielsweise aus Fibrella mit denselben Abmessungen wie die erste äußere Lage wird vorsichtig auf die gekreuzten Lagen der Garnabschnitte aufgelegt und mit einem handelsüblichen Schaumstoffroller waagrecht und diagonal abgerollt, bzw. angedrückt, so dass die beiden äußeren Lagen durch die dazwischen befindlichen gekreuzten Lagen der mit Leim versehenen Garnabschnitte miteinander verbunden und verklebt werden. Zwischen den einzelnen Garnabschnitten bilden sich geschlossene Kassetten, in denen sich jeweils eine gleichgroße Portion des superabsorbierenden Polymermaterials befindet.

Eine Zuschneideplatte mit den Abmessungen 220 mm x 500 mm wird auf das in dieser Weise hergestellte absorbierende Laminat aufgelegt und das Laminat auf diese Größe zugeschnitten, wobei darauf geachtet werden muss, dass keine Kassetten mit superabsorbierendem Polymermaterial angeschnitten werden und kein superabsorbierendes Polymermaterial austritt.

Um mit diesem absorbierenden Laminat ein Testmuster eines windelartigen saugfähigen Hygieneprodukts herzustellen werden auf einer ersten Metallplatte im Abstand von 125 mm zwei Metallschienen befestigt. Auf den beiden Metallschienen sind jeweils sechs Metallstifte angebracht, um die ein elastisches Garn (615 dTex, 550 den, Dorlastan) spannungsfrei gelegt wird, wobei sich folgendes Muster parallelverlaufender Fäden des Garns ergibt. Es wird eine erste Gruppe von 3 parallelen Fäden mit einem Abstand von 5 mm zueinander gebildet, im Abstand von 50 mm gefolgt von einer zweiten Gruppe von 2 parallelen Fäden, wobei die 2 Fäden in dieser Gruppe auch jeweils einen Abstand von 5 mm zueinander aufweisen. In einem Abstand von 65 mm folgt eine dritte Gruppe, wiederum gebildet aus 2 parallelen Fäden, die jeweils in einem Abstand von 5 mm zueinander verlaufen. Das Fadenmuster wird durch eine vierte Gruppe aus 3 parallelen Fäden in einem Abstand von 5 mm zueinander komplementiert. Die vierte Gruppe folgt ihrerseits in einem Abstand von 50 mm auf die dritte Gruppe. Der Abstand von Gruppe 1 zu Gruppe 4 beträgt hierbei insgesamt 190 mm. Die beiden Metallschienen werden abgenommen und auf einer zweiten Metallplatte von 400 mm x 600 mm mit einem Abstand von 520 mm zueinander festgelegt, wobei dadurch die Fäden vorgespannt werden. Unter die Fadengruppe 1 und 2 sowie 3 und 4 wird jeweils ein Streifen aus Corovin (160 mm x 520 mm) so auf die Metallplatte aufgelegt, dass die Längsrichtung der beiden Streifen aus Corovin mit der Ausrichtung der Fadengruppen übereinstimmt und die Mitte in Längsrichtung eines jeden Corovin-Streifens unterhalb der inneren zugeordneten Fadengruppe 2 bzw. 3 ausgerichtet ist. Die beiden Corovin-Streifen überlappen sich dabei zwischen den inneren Fadengruppen. Die Garnabschnitte werden an den für die Verleimung der Garnfäden relevanten Stellen mit Leim besprüht (Leimpistole HB 700 von Bühnen mit einer Hotmelt-Düse von Bühnen, Druck etwa 2,5 bar, Leim Bostik 2052 FUN von Bostik). Anschließend wird nacheinander jeder der Corovin-Streifen ebenfalls mit Leim besprüht, entlang der Mitte in Längsrichtung von innen nach außen gefaltet, so dass die gefalteten Hälften und die beiden nunmehr außen befindlichen Längskanten eines jeden Corovin-Streifens deckungsgleich übereinander zu liegen kommen und jeder gefaltete Corovin-Streifen die ihm zugeordneten Fadengruppen 1 und 2 bzw. 3 und 4 umschließt. Die übereinanderliegenden Hälften der Corovin-Streifen werden aneinander angedrückt und miteinander verklebt.

Die gefalteten Corovin-Streifen werden mit Leim besprüht und etwas auseinandergezogen, so dass die einander zugewandten Längskanten einen Abstand von 90 mm zueinander aufweisen. Anschließend wird ein Topsheet (170 mm x 520 mm, Novellin) aufgelegt und längs der Seitenkanten auf die Corovin-Streifen aufgedrückt, bzw. mit diesen verklebt.

Der mittlere Bereich des Topsheets wird entlang des späteren Randbereichs des darauf zu verklebenden absorbierenden Laminats verleimt und innerhalb des Randbereichs vernebelt verleimt. Die später dem topsheet zugewandte äußere Lage des absorbierenden Laminats wird ebenfalls verleimt. In den mittleren Bereich des Topsheets wird das absorbierende Laminat aufgelegt, wobei das absorbierende Laminat auf 400 mm X 120 mm auseinandergezogen und mit der dem topsheet zugewandten äußeren Lage auf das Topsheet aufgelegt wird.

Anschließend erfolgt eine Verleimung des absorbierenden Laminats, der Endlaschen und eine Randverleimung. Zum Schluss wird das Backsheet (Folie RKW Hypor B 140 textile, Typ 45755 weiß mit Abmessungen 210 mm x 520 mm) aufgelegt und fixiert, bzw. mit einem Schaumstoffroller angedrückt.

Für die Durchführung der Versuche wurden eine Anzahl von Testmustern saugfähiger Hygieneprodukte hergestellt, die sich jeweils nur durch das Vliesmaterial der äußeren Lagen des absorbierenden Laminats unterscheiden. Es wurden jeweils 4 gleichartige Testmuster für jede untersuchte Kombination von Vliesmaterialien für die äußeren Lagen des absorbierenden Laminats hergestellt.

Zur Bestimmung der Einsickerzeit wurde jeweils vier Mal nacheinander mit einem zeitlichen Abstand eine Menge von 70 ml einer 0,9%igen Kochsalzlösung innerhalb von 10 s durch einen Trichter mittig auf dem Topsheet eines jeden Testmusters des saugfähigen Hygieneprodukts aufgebracht. Der Trichter besteht aus einer Makrolonplatte (400 mm x 300 mm x 10 mm) mit einem mittig angeordneten Einfüllstutzen (44 mm Innendurchmesser, 80 mm hoch) und einem Gesamtgewicht von 940 g). Der Trichter wurde an gegenüberliegenden Stirnseiten mit zwei rechteckigen Gewichten mit einem Gewicht von jeweils 3800 g beschwert.

Die Einsickerzeit wird als diejenige Zeitdauer bestimmt, bis die Flüssigkeit nach erfolgter vollständiger Einfüllung in dem Testmuster komplett eingesickert ist und sich keine Flüssigkeit mehr im Einfüllstutzen befindet.

Falls während oder nach einer der vier aufeinanderfolgenden Flüssigkeitszugaben an irgendeiner Stelle des saugfähigen Hygieneprodukts Flüssigkeit austritt, wird der Test abgebrochen. In diesem Fall wird das betreffende Testmuster als ungeeignet angesehen, da die Flüssigkeit nicht vollständig aufgenommen und zurückgehalten werden konnte.

Zur Bestimmung der Rücknässung werden jeweils 20 Minuten nach jeder Flüssigkeitszugabe auf beiden Seiten in einem Abstand von 8 cm zur Mitte des Testmusters jeweils ein mehrlagiger Stapel Filterpapier mit einem Gesamtgewicht von mindestens 3,5 g aufgelegt und jeweils mit einem runden Gewicht von 1200 g beschwert. Bei dem Filterpapier handelt es sich um runde Filterpapiere Macherey-Nagel, MN 617, mit einem Durchmesser von 90 mm. Nach 2 min werden die Gewichte und die Filterpapierstapel entnommen. Die Rücknässung wird als Gesamtsumme "Gesamte Rücknässung" des beiden Differenzgewichte "Rücknässung 1" und "Rücknässung 2" der beiden Filterpapierstapel nach der gewichtsbeschwerten Auflagedauer von 2 min auf dem flüssigkeitsgefüllten saugfähigen Hygieneprodukt abzüglich des Trockengewichts des Filterpapierstapels bestimmt.

Die jeweils für mindestens 4 gleichartige Testmuster durchgeführten Versuche und Messungen ergaben im Durchschnitt folgende Ergebnisse für die Einsickerzeit und die Rücknässung nach der vierten und letzten Flüssigkeitszugabe:

| topsheet-seitige äußere Lage | backsheet-seitige äußere Lage | Einsicker zeit [s] | Rücknässung 1 [g] | Rücknässung 2 [g] | Rücknässung insgesamt [g] | Flüssigkeitsaustritt |
|---|---|---|---|---|---|---|
| Fibrella | Fibrella | 47,0 | 3,19 | 0,70 | 3,89 | nein |
| Novellin | Novellin | 42,0 | 3,10 | 1,76 | 4,86 | nein |
| Corovin | Corovin | - | - | - | - | ja |
| Pegas | Pegas | - | - | - | - | ja |
| Fibrella | Corovin | 38,0 | 2,15 | 4,31 | 6,28 | nein |
| Corovin | Fibrella | 30,7 | 0,11 | 0,11 | 0,22 | nein |

Diese Versuche zeigen, dass erwartungsgemäß bei der Verwendung eines hydrophilen Vliesmaterials wie beispielsweise Fibrella oder Novellin für die beiden äußeren Lagen des Laminats eine rasche Flüssigkeitsaufnahme (kurze Einsickerzeit im Bereich von etwa 45 s) erreicht wird. Allerdings bieten die hydrophilen Vliesmaterialien keinen überzeugenden Schutz vor einer Rücknässung, weshalb jeweils etwa 3,9 g bzw. 4,9 g als Differenzgewicht für die gesamte Rücknässung ermittelt wurden.

Die untersuchten hydrophoben Vliesmaterialien wie beispielsweise Corovin oder Pegas führten jeweils dazu, dass während oder unmittelbar nach einer Flüssigkeitszugabe Flüssigkeit ausgetreten ist. Ein saugfähiges Hygieneprodukt mit einem derartigen absorbierenden Laminat, bzw. mit zwei äußeren Lagen aus diesen hydrophoben Vliesmaterialien erscheint nicht geeignet und kann im Gegensatz zu anderen Vliesmaterialien einen unerwünschten Flüssigkeitsaustritt nicht verhindern.

Auch die Kombination einer topsheet-seitigen äußeren Lage aus einem hydrophilen Material (beispielsweise Fibrella) und einer backsheet-seitigen äußeren Lage aus einem hydrophoben Material (beispielsweise Corovin) führt nicht zu überzeugenden Ergebnissen. Die Einsickerzeit ist mit 38 s vergleichsweise kurz, jedoch tritt eine sehr hohe Rücknässung mit etwa 6,3 g Flüssigkeitsaufnahme in dem Filterpapier auf.

Überraschenderweise haben die Versuche ergeben, dass die Kombination einer topsheet-seitigen äußeren Lage aus einem hydrophoben Material (beispielsweise Corovin) und einer backsheet-seitigen äußeren Lage aus einem hydrophilen Material (beispielsweise Fibrella) die besten Ergebnisse liefert. Trotz des hydrophoben Vliesmaterials, das für die topsheet-seitige äußere Lage des absorbierenden Laminats verwendet wird, kann eine sehr rasche Einsickerzeit von etwa 31 s erreicht werden. Das rasche Einsickern wird durch die Struktur des absorbierenden Laminats, bzw. durch die bei der vorangehend beschriebenen Herstellung der Laminate gebildeten Transportkanäle zwischen den einzelnen Kassetten oder Waben mit superabsorbierendem Polymermaterial begünstigt. Zudem beträgt die gesamte Rücknässung lediglich 0,22 g und ist damit um mehr als eine Größenordnung besser als alle anderen Werte der Rücknässung für andere Kombinationen von Vliesmaterialien. Die erfindungsgemäße Kombination und Anordnung der vorangehend beschriebenen Auswahl eines hydrophoben Vliesmaterials zum Topsheet hin und des hydrophilen Vliesmaterials zum Backsheet hin ergibt im Vergleich mit allen anderen Materialkombinationen die kürzeste Einsickerzeit in Verbindung mit einer um eine Größenordnung geringeren Rücknässung.

Ein exemplarisches Ausführungsbeispiel des Erfindungsgedankens wird anhand der Figuren näher erläutert. Es zeigt:
Fig. 1 eine schematische Draufsicht auf ein erfindungsgemäßes saugfähiges Hygieneprodukt, und
Fig. 2 eine Schnittansicht des in Fig. 1 gezeigten saugfähigen Hygieneprodukts längs der Linie II-II in Fig. 1.

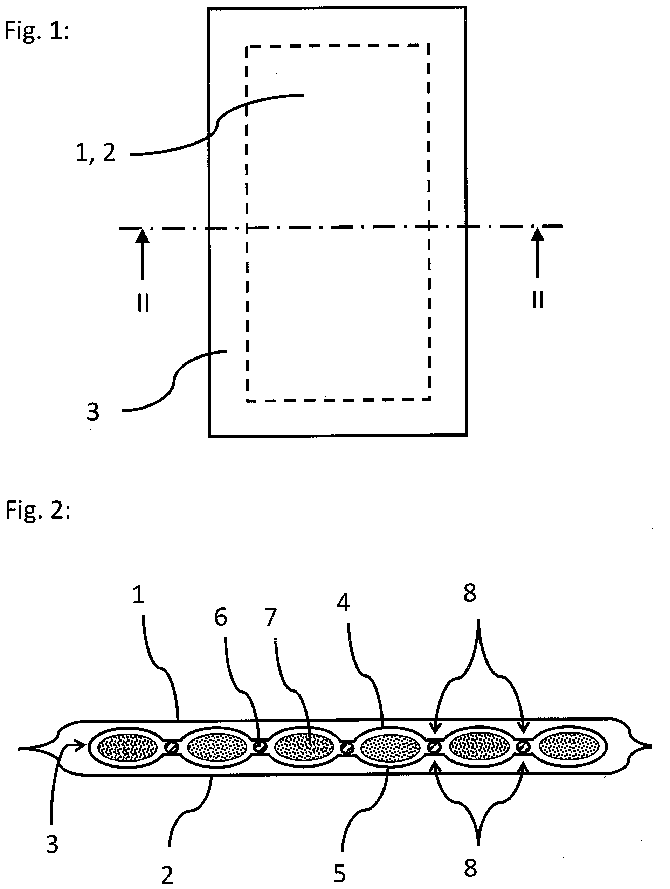

Ein exemplarisch in den Fig. 1 und 2 dargestelltes saugfähiges Hygieneprodukt weist eine erste äußere Lage, ein Topsheet (1) auf, welche der Hautseite eines Trägers zugewandt ist und üblicherweise aus einem hydrophilen Material besteht. Es weist ferner eine weitere äußere Lage, ein Backsheet (2) auf, welches gegenüberliegend der Kleidungsseite des Trägers zugewandt ist und üblicherweise aus einem hydrophoben Material besteht.

Zwischen dem Topsheet (1) und dem Backsheet (2) ist ein absorbierendes Laminat (3) eingelagert. Das Laminat (3) kann haftend mit dem Topsheet (1) und/oder mit dem Backsheet (2) des Produktes verbunden sein, wobei diese Verbindung ebenso wie die Verbindung von Topsheet (1) und Backsheet (2) in einem das Laminat (3) umgebenden Randbereich miteinander wahlweise flächig, punktuell oder linienförmig mittels Haftkleber, Verschweißung, Vernadelung oder anderen geeigneten Befestigungsverfahren oder Befestigungsmitteln erfolgt.

Es kann für bestimmte Anwendungen auch vorteilhaft sein, lediglich das Topsheet (1) und das Backsheet (2) längs eines umlaufenden Randes mindestens abschnittsweise miteinander zu verbinden und zu befestigen und das absorbierende Laminat (3) lose einzulegen oder lediglich an dem Backsheet (2) festzulegen, um die Flüssigkeitsaufnahme durch das Topsheet (1) nicht zu behindern und eine größtmögliche Quellung des Laminats (3) in alle Richtungen zu erlauben. Es ist ebenso denkbar, dass das absorbierende Laminat (3) zur Backsheet-Seite hin mindestens bereichsweise lose an dem Backsheet (2) anliegt.

Dieses absorbierende Laminat (3) besteht aus zwei biegeweichen äußeren Lagen (4, 5), welche mit zwei dazwischen angeordneten und sich diagonal kreuzenden Lagen aus vorgespannten elastischen Fäden oder Bänder (6) haftend miteinander verbunden sind. Das Laminat (3) weist eine steppdeckenartige Struktur auf, in deren offenen Kassetten superabsorbierende Granulate oder Fäden (7) eingelagert sind, die üblicherweise aus einem superabsorbierenden Polymer bestehen.

Ausführungsformen und Herstellungsmethoden dieses Laminats (3), das den Absorptionskern bildet, sind beispielsweise in DE 10 2010 013 288.8 beschrieben, deren Inhalt vollständig in diese Beschreibung mit aufgenommen wird.

Dieser Absorptionskern kann sowohl flächendeckend mit dem Topsheet (1) und/oder Backsheet (2) des saugfähigen Hygieneprodukts verhaftet sein, als auch in Querrichtung der Herstellung des saugfähigen Hygieneprodukts schmaler oder breiter ausgeführt sein oder in Längsrichtung der Herstellung in diskreten Einzelstücken aufgetragen werden ("Cut&Space").

An oder in dem saugfähigen Hygieneprodukt können wahlweise elastische Verschlusssysteme, elastifizerte Elemente für die Optimierung der Passform, zusätzliche Komponenten zur Verbesserung der Handhabung, externe Barrieren zur Kontrolle der Körperflüssigkeiten, etc. angeordnet und festgelegt werden.

Erfinderisch und kennzeichnend für den Aufbau des saugfähigen Hygieneprodukts ist die teilweise Umkehr des klassischen Modells der absorbierenden Hygieneartikel "Aufnahme-Verteilung-Speicherung" und die Verlagerung eines Teils der Flüssigkeitsverteilungsfunktion von dem hautseitigen Topsheet (1) hin zu dem bekleidungsseitigen Backsheet (2).

Dies wird erreicht, indem die dem Topsheet (1) zugewandte äußere Lage (4) des Laminats (3) aus einem biegeweichen hydrophoben Material gebildet wird, welches sektionsweise oder punktuell durchlässig für Flüssigkeiten gemacht wird. Dies geschieht bevorzugt durch Wärme, Druck, mechanische Penetration, Wechselwirkung mit dem Haftkleber oder durch andere geeignete Verfahren, mit denen das hydrophobe Material bereichsweise für Flüssigkeit durchlässig gemacht werden kann. Ein Beispiel hierfür ist Corovin.

Die dem Backsheet (2) zugewandte äußere Lage (5) des Laminats (3) wird dagegen erfindungsgemäß aus einem biegeweichen Material mit hoher Transportfähigkeit für Flüssigkeiten gebildet. Geeignet sind hier besonders Vliesstoffe, welche typischerweise als Materialien für Wet Wipes oder als Acquisition/Distribution Layer (ADL) in Babywindeln eingesetzt werden, z.B. spunlace PET, PET-Viskose, Viskose, PP Vliese, cardierte, thermobondierte hydrophile Polypropylenvliese, Hygienepapier oder vergleichbare bekannte Materialien. Ein Beispiel hierfür ist Fibrella.

Es ist vorteilhaft, diese Durchlässigkeit gezielt in den Sektionen zur erzeugen, in denen die dem Topsheet (1) zugewandte äußere Lage (4) und die dem Backsheet (2) zugewandte äußere Lage (5) mit den elastischen Fäden (6) miteinander haftend verbunden sind, so das sich in diesen durch die elastischen Fäden (6) unterteilten Sektionen ein gewünschter Kapillareffekt durch einerseits eine feine Lochstruktur der dem Topsheet (1) zugewandten Lage (4) und andererseits eine Sogwirkung durch die Flüssigkeitstransporteigenschaften der dem Backsheet (2) zugewandten äußeren Lage (5) des Laminats (3) ergibt. Dadurch gebildete offene Kassetten des Laminats (3) sind hautseitig durch die dem Topsheet (1) zugewandte äußere Lage (4) weiterhin im Wesentlichen hydrophob abgedeckt, so das sich hier eine gewisse strukturelle Barriere der Rücknässung zur Hautseite ergibt.

Es ist ferner vorteilhaft, dass sich durch die Kassettenform des Laminats (3) sowohl zwischen der dem Topsheet (1) zugewandten äußeren Lage (4) und dem Topsheet(1) als auch speziell zwischen der dem Backsheet (2) zugewandten äußeren Lage (5) und dem Backsheet(2) eine Vielzahl von offenen Transportkanälen (8) bildet, welche für eine schnelle Verteilung der Flüssigkeit in Längs- und Querrichtung des saugfähigen Hygieneprodukts sorgen. Die Kanalbildung wird durch die Quellung des Laminats (3) noch unterstützt, so das im Gegensatz zu gängigen absorbierenden Hygieneprodukten die Flüssigkeitsverteilfunktion des saugfähigen Hygieneprodukts mit zunehmender Menge an absorbierter Flüssigkeit sich nicht verschlechtert, sondern tendenziell verbessert.

Der Eintrag der Flüssigkeit in den Superabsorber des Laminats (3) wird unterstützt durch die Transportfähigkeit der bekleidungsseitigen, dem Backsheet (2) zugewandten äußeren Lage (5) des Laminats (3), welches die Flüssigkeitsverteilung in den backsheet-seitigen Transportkanälen (8) unterstützt und in vertikaler Richtung die Flüssigkeit an den Superabsorber weiterleitet.

## Patentansprüche

1. Saugfähiges Hygieneprodukt zur Aufnahme von Flüssigkeiten, gebildet zumindest aus einem biegeweichen Topsheet (1) und einem biegeweichen Backsheet (2), zwischen denen haftend ein absorbierendes Laminat (3) eingebracht ist, bestehend aus zwei biegeweichen äußeren Lagen (4, 5), zwischen denen mindestens zwei sich diagonal kreuzende, vorgespannte Lagen von elastischen Fäden (6) haftend eingelagert sind und in welches diskrete Sektionen von superabsorbierendem Polymer (7) eingelagert sind, wobei die topsheet-seitige äußere Lage (4) des Laminats (3) ein hydrophobes Material ist, welches im Bereich der Verhaftung mit den elastischen Fäden (6) und der zweiten äußeren Lage (5) punktuell oder sektionsweise mechanisch, chemisch oder thermisch flüssigkeitsdurchlässig gemacht wurde,
wobei das Laminat (3) kassettenförmige Bereiche mit darin angeordnetem superabsorbierenden Polymer (7) aufweist,
und wobei zwischen den kassettenförmigen Bereichen des Laminats (3) eine Vielzahl von offenen Transportkanälen (8) ausgebildet sind.

2. Saugfähiges Hygieneprodukt nach Anspruch 1, **dadurch gekennzeichnet, dass** die topsheet-seitige äußere Lage (4) des Laminats (3) nach 300 s Flüssigkeitszugabe eine Sperrwirkung von mehr als 2 cm für Flüssigkeiten aufweist, wobei die Sperrwirkung nach der in Beispiel 1 niedergelegten Messmethode bestimmt ist.

3. Saugfähiges Hygieneprodukt nach Anspruch 2, **dadurch gekennzeichnet, dass** die topsheet-seitige äußere Lage (4) des Laminats (3) nach 300 s Flüssigkeitszugabe eine Sperrwirkung von mehr als 5 cm, bevorzugt mehr als 8 cm und besonders bevorzugt mehr als 10 cm für Flüssigkeiten aufweist, wobei die Sperrwirkung nach der in Beispiel 1 niedergelegten Messmethode bestimmt ist.

4. Saugfähiges Hygieneprodukt nach einem der Ansprüche 1 bis 3, **dadurch gekennzeichnet, dass** die backsheet-seitige äußere Lage (5) des Laminats (3) ein hydrophiles Material mit guter Flüssigkeitstransporteigenschaft ist.

5. Saugfähiges Hygieneprodukt nach Anspruch 4, **dadurch gekennzeichnet, dass** die backsheet-seitige äußere Lage (5) des Laminats (3) ein hydrophiles Material mit einer Flächenverteilung von mehr als 10 cm² benetzte Fläche durch eine punktförmig aufgebrachte Flüssigkeitsmenge von 1 ml ist, wobei die Flächenverteilung nach der in Beispiel 2 niedergelegten Messmethode bestimmt ist.

6. Saugfähiges Hygieneprodukt nach Anspruch 4, **dadurch gekennzeichnet, dass** die backsheet-seitige äußere Lage (5) des Laminats (3) aus einem hydrophilen Material mit einer Flächenverteilung von mehr als 20 cm², bevorzugt mehr als 40 cm² und besonders bevorzugt mehr als 55 cm² benetzte Fläche durch eine punktförmig aufgebrachte Flüssigkeitsmenge von 1 ml ist, wobei die Flächenverteilung nach der in Beispiel 2 niedergelegten Messmethode bestimmt ist.

7. Saugfähiges Hygieneprodukt nach einem der vorangehenden Ansprüche, **dadurch gekennzeichnet, dass** das absorbierende Laminat (3) zur Topsheet-Seite hin mindestens bereichsweise lose an dem Topsheet (1) anliegt.

8. Saugfähiges Hygieneprodukt nach einem der vorangehenden Ansprüche, **dadurch gekennzeichnet, dass** das absorbierende Laminat (3) zur Backsheet-Seite hin mindestens bereichsweise lose an dem Backsheet (2) anliegt.

## Claims

1. Absorptive hygiene product for absorbing liquids, formed at least from a flexible topsheet (1) and a flexible backsheet (2), between which there has been inserted and bonded an absorptive laminate (3) consisting of two flexible outer plies (4, 5), between which there have been incorporated and bonded at least two diagonally crossing, pretensioned plies of elastic filaments (6) and into which discrete sections of superabsorbent polymer (7) have been incorporated, wherein the topsheet-side outer ply (4) of the laminate (3) is a hydrophobic material that has been rendered liquid-permeable by mechanical, chemical or thermal means, in a punctiform manner or in sections, in the region of the adhesion to the elastic filaments (6) and the second outer ply (5), wherein the laminate (3) has regions in the form of cassettes with superabsorbent polymer (7) disposed therein,
and wherein a multitude of open transport channels (8) are formed between the regions of laminate (3) in the form of cassettes.

2. Absorptive hygiene product according to Claim 1, **characterized in that** the topsheet-side outer ply (4) of the laminate (3), after addition of liquid for 300 s, has a barrier action of more than 2 cm for liquids, wherein the barrier action is determined by the test method set out in example 1.

3. Absorptive hygiene product according to Claim 2, **characterized in that** the topsheet-side outer ply (4) of the laminate (3), after addition of liquid for 300 s, has a barrier action of more than 5 cm, preferably more than 8 cm and more preferably more than 10 cm for liquids, wherein the barrier action is determined by the test method set out in example 1.

4. Absorptive hygiene product according to any of Claims 1 to 3, **characterized in that** the backsheet-side outer ply (5) of the laminate (3) is a hydrophilic material having good liquid transport capacity.

5. Absorptive hygiene product according to Claim 4, **characterized in that** the backsheet-side outer ply (5) of the laminate (3) is a hydrophilic material having an area distribution of more than 10 cm² of wetted area by an amount of liquid of 1 ml applied in a punctiform manner, wherein the area distribution is determined by the test method set out in example 2.

6. Absorptive hygiene product according to Claim 4, **characterized in that** the backsheet-side outer ply (5) of the laminate (3) is a hydrophilic material having an area distribution of more than 20 cm², preferably more than 40 cm² and more preferably more than 55 cm² of wetted area by an amount of liquid of 1 ml applied in a punctiform manner, wherein the area distribution is determined by the test method set out in example 2.

7. Absorptive hygiene product according to any of the preceding claims, **characterized in that** the absorbing laminate (3) loosely adjoins the topsheet (1) at least in regions on the topsheet side.

8. Absorptive hygiene product according to any of the preceding claims, **characterized in that** the absorbing laminate (3) loosely adjoins the backsheet (2) at least in regions on the backsheet side.

## Revendications

1. Produit hygiénique absorbant destiné à recevoir des liquides, formé par au moins une feuille supérieure souple à la flexion (1) et une feuille inférieure souple à la flexion (2), entre lesquelles un stratifié absorbant (3) est introduit de manière adhérente, constitué par deux couches extérieures souples en flexion (4, 5), entre lesquelles au moins deux couches précontraintes de fils élastiques (6), se croisant en diagonale, sont insérées de manière adhérente, et dans lequel des sections discrètes de polymère superabsorbant (7) sont insérées, la couche extérieure (4) du stratifié (3), côté feuille supérieure, étant un matériau hydrophobe, qui a été rendu perméable aux liquides mécaniquement, chimiquement ou thermiquement, ponctuellement ou en sections, dans la zone de l'adhésion avec les fils élastiques (6) et la deuxième couche extérieure (5),
le stratifié (3) comprenant des zones en forme de cassettes dans lesquelles est agencé du polymère superabsorbant (7),
et une pluralité de canaux de transport ouverts (8) étant formés entre les zones en forme de cassettes du stratifié (3).

2. Produit hygiénique absorbant selon la revendication 1, **caractérisé en ce que** la couche extérieure (4) du stratifié (3), côté feuille supérieure, présente après 300 s d'ajout de liquide un effet de barrière de plus de 2 cm pour les liquides, l'effet de barrière étant déterminé selon la méthode de mesure définie dans l'exemple 1.

3. Produit hygiénique absorbant selon la revendication 2, **caractérisé en ce que** la couche extérieure (4) du stratifié (3), côté feuille supérieure, présente après 300 s d'ajout de liquide un effet de barrière de plus de 5 cm, de préférence de plus de 8 cm et de manière particulièrement préférée de plus de 10 cm, pour les liquides, l'effet de barrière étant déterminé selon la méthode de mesure définie dans l'exemple 1.

4. Produit hygiénique absorbant selon l'une quelconque des revendications 1 à 3, **caractérisé en ce que** la couche extérieure (5) du stratifié (3), côté feuille inférieure, est un matériau hydrophile présentant de bonnes propriétés de transport de liquide.

5. Produit hygiénique absorbant selon la revendication 4, **caractérisé en ce que** la couche extérieure (5) du stratifié (3), côté feuille inférieure, est un matériau hydrophile présentant une distribution de surface de plus de 10 cm² de surface mouillée par une quantité de liquide appliquée ponctuellement de 1 ml, la distribution de surface étant déterminée selon la méthode de mesure définie dans l'exemple 2.

6. Produit hygiénique absorbant selon la revendication 4, **caractérisé en ce que** la couche extérieure (5) du stratifié (3), côté feuille inférieure, est un matériau hydrophile présentant une distribution de surface de plus de 20 cm², de préférence de plus de 40 cm² et de manière particulièrement préférée de plus de 55 cm², de surface mouillée par une quantité de liquide appliquée ponctuellement de 1 ml, la distribution de surface étant déterminée selon la méthode de mesure définie dans l'exemple 2.

7. Produit hygiénique absorbant selon l'une quelconque des revendications précédentes, **caractérisé en ce que** le stratifié absorbant (3) repose, dans la direction du côté de la feuille supérieure, au moins en sections de manière lâche sur la feuille supérieure (1) .

8. Produit hygiénique absorbant selon l'une quelconque des revendications précédentes, **caractérisé en ce que** le stratifié absorbant (3) repose, dans la direction du côté de la feuille inférieure, au moins en sections de manière lâche sur la feuille inférieure (2) .
